# EUROPEAN PATENT APPLICATION

(11) **EP 1 072 261 A2**
(43) Date of publication of application: **31.01.2001**
(21) Application number: 00116436.7
(22) Date of filing: 28.07.2000
(51) Int. Cl.: A61K 9/70, A61K 31/192

(54) **Felbinac-containing patch**

(30) Priority: 30.07.1999 JP 21731399
(71) Applicant: HISAMITSU PHARMACEUTICAL CO., INC., Tosu-shi Saga-ken (JP)
(72) Inventor: Takada, Yasunori, c/o Hisamitsu Pharmac. Co., Inc., Tosu-shi, Saga 841-0017 (JP); Ikeura, Yasuhiro, c/o Hisamitsu Pharmac. Co., Inc., Tosu-shi, Saga 841-0017 (JP); Tanaka, Koji, c/o Hisamitsu Pharmaceutic. Co.,Inc., Tosu-shi, Saga 841-0017 (JP)
(74) Representative: Grünecker, Kinkeldey, Stockmair & Schwanhäusser Anwaltssozietät

(57) **Abstract**

There is provided a felbinac-containing patch that stably produces a sufficient anti-inflammatory, analgesic effect by felbinac exhibiting a strong anti-inflammatory, analgesic action without bringing harmful effects such as skin irritation over a long period and, at the same time, that is excellent in the stability of preparations as well as in their adhesive properties, the felbinac-containing patch comprising 10-30 wt% of a styrene-isoprene-styrene block copolymer, 5-15 wt% of polyisobutylene, 5-30 wt% of a rosin-based resin, 30-70 wt% of a plasticizer, 1-5 wt% of felbinac, and 0.01-5 wt% of a dispersing agent,
wherein the drug release rate of the patch at one hour after the start of the water-release test using a rotating cylinder described in the release test method as prescribed in United State Pharmacopoeia is 20±15 wt%, the drug release rate of the patch at three hours after the start of test is 40±15 wt%, and the drug release rate of the patch at six hours after the start of test is 60±20 wt%.

## Description

### BACKGROUND OF THE INVENTION

### Field of the Invention

The present invention relates to a felbinac-containing patch (plaster) for external application that is intended for anti-inflammatory, analgesic effect. More particularly, it relates to a patch containing felbinac (generic name: 4-biphenylacetic acid), which is a non-steroidal anti-inflammatory, analgesic agent, as a medicinally effective component for the purpose of treating lumbago, myalgia, periarthritis, and so on.

### Related Background Art

Felbinac (4-biphenylacetic acid) is an active metabolite of fenbufen, a non-steroidal anti-inflammatory, analgesic agent and is a drug exhibiting a strong anti-inflammatory, analgesic action. Since this drug is not suitable for oral administration, attention has been focused on studies of preparations for percutaneous administration. Gels, liquids, and gel patches (poultices) containing the above-mentioned drug have hitherto been commercially available. The gels and liquids, however, had problems such as difficulty in administration in given quantities, low bioavailability, adhesion to clothing, and the frequency of administration (e.g., several times of administration per day). The gel patches have been developed in order to overcome these problems with the gels and liquids, but they still had problems such as the incapability of being affixed for a long period of time because of their weak adhesive strength, low bioavailability, and insufficient duration of their efficacy.

Japanese Patent Application Laid-Open Gazette No. Hei. 4-321624 proposed an anti-inflammatory, analgesic patch which employed a styrene-isoprene-styrene block copolymer as the principle component of the base and crotamiton as an essential solubilizer. International Publication WO98/24423 proposed an anti-inflammatory, analgesic patch which contains a styrene-isoprene-styrene block copolymer, a rosin-type resin, a plasticizer, and felbinac but which contains no crotamiton.

### SUMMARY OF THE INVENTION

The present inventors, however, found that the anti-inflammatory, analgesic patches described in the Laid-Open Gazette No. Hei. 4-321624 and International Publication WO98/24423 were not yet satisfactory, as will be described below. Namely, the anti-inflammatory, analgesic patch described in Laid-Open Gazette No. Hei. 4-321624 above was not yet satisfactory: work steps were cumbersome because crotamiton was used as a solubilizer for the drug; time-dependent decrease in adhesive strength occurred due to bleeding (percolating to the surface) of crotamiton; and there was poor adhesion to flexion sites such as elbow and knee. Also, the anti-inflammatory, analgesic patch described in WO98/24423 was not yet satisfactory in the duration of efficacy (anti-inflammatory, analgesic effect): the drug release is high to some extent despite that it does not contain any crotamiton as the solubilizer; however, the constantly high drug release is not maintained stably.

The present invention has been made in view of the problems of the prior art described above. An object of the invention is to provide a felbinac-containing patch that can stably produce, over a long period of time, a sufficient anti-inflammatory, analgesic effect by felbinac, which exhibits a strong anti-inflammatory, analgesic action, without bringing harmful effects such as skin irritation, and that is excellent in the stability of preparations as well as in their adhesive properties.

The present inventors conducted extensive studies to achieve the above-stated object. Consequently, the inventors found that the sufficient anti-inflammatory, analgesic effect is produced stably for a long period of time without bringing harmful effects by such felbinac-containing patch that its release rates of felbinac, the medicinally component, one hour, three hours, and six hours later are in a predetermined range and also found that the felbinac-containing patch can be obtained by including specific components in specific proportions, thus accomplishing the present invention.

Specifically, a felbinac-containing patch of the present invention comprises 1-5 wt% of felbinac as a medicinally effective component,
characterized in that the drug release rate of the patch at one hour after the start of test is 20±15 wt%, the drug release rate of the patch at three hours after the start of test is 40±15 wt%, and the drug release rate of the patch at six hours after the start of test is 60±20 wt%, when the water-releasing test using a rotating cylinder described in the release test method as prescribed in United State Pharmacopoeia is carried out under the following conditions:
Test solution: Solution 2 described in the disintegration test method as prescribed in The Pharmacopoeia of Japan
Solution temperature: 37±0.5 °C
The distance between the lower end of cylinder and the inner surface of container: 12±2 mm
The number of rotations: 50 rpm.

Preferably, the felbinac-containing patch of the invention comprises 10-30 wt% of a styrene-isoprene-styrene block copolymer, 5-15 wt% of polyisobutylene, 5-30 wt% of a rosin-based resin, 30-70 wt% of a plasticizer, 1-5 wt% of felbinac, and 0.01-5 wt% of a dispersing agent. The dispersing agent is preferably synthetic aluminum silicate and/or hydrated aluminum silicate.

Moreover, the felbinac-containing patch of the invention described above is preferably further provided with a backing. In this case, it is preferred that the backing show a load at 30%-elongation of 100-800 g in the longitudinal direction and a load at 30%-elongation of 500-2500 g in the lateral direction as well as show a recovery factor at 50%-elongation of 75-95% in the longitudinal direction and a recovery factor at 50%-elongation of 65-85% in the lateral direction, under such measurement conditions that the sample width is 50 mm, the sample length is 200 mm, and the elongation speed is 200 mm/min.

According to the present invention, it will be possible to obtain the felbinac-containing patch that stably produces a sufficient anti-inflammatory, analgesic effect by felbinac exhibiting a strong anti-inflammatory, analgesic action without bringing harmful effects such as skin irritation over a long period of time and, at the same time, that is excellent in the stability of preparations as well as in their adhesive properties.

Accordingly, the present invention can provide the felbinac-containing patch useful as a medicine (patch for external application) that is intended for anti-inflammatory, analgesic effect.

The present invention will be more fully understood from the detailed description given hereinbelow and the accompanying drawings, which are given by way of illustration only and are not to be considered as limiting the present invention.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will be apparent to those skilled in the art from this detailed description.

### BRIEF DESCRIPTION OF THE DRAWINGS

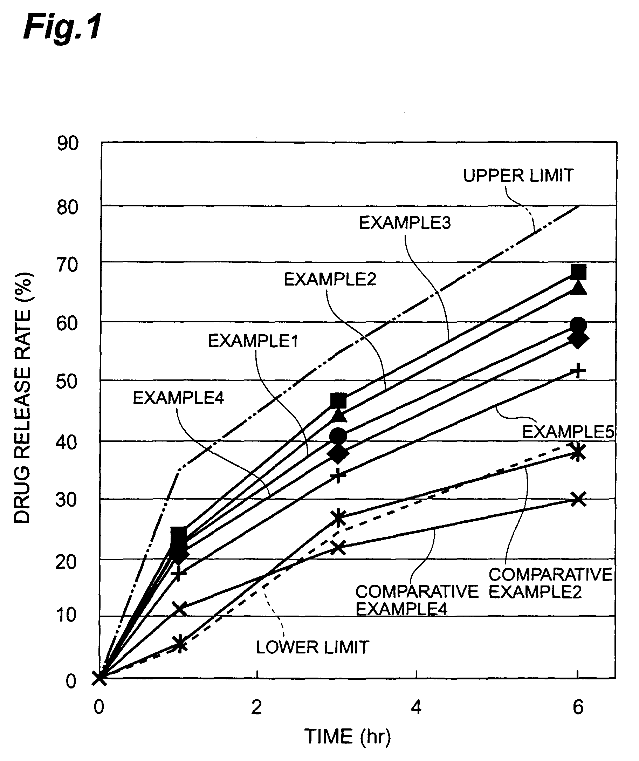

Fig. 1 is a graph showing the results of drug release test.

### DESCRIPTION OF THE PREFERRED EMBODIMENTS

The felbinac-containing patch of the present invention comprises as a medicinally effective component, 1-5 wt%, preferably 2-4 wt%, of felbinac (i.e., 4-biphenyl acetic acid). By employing this blending ratio, good percutaneous absorption of the drug, duration of the efficacy, dispersibility of the drug and so on, besides being economically excellent, will be achieved. Here, if the blending ratio of felbinac is less than 1 wt%, sufficient efficacy will not be attained. On the other hand, blending ratios over 5 wt% will result in the occurrence of harmful effects such as skin irritation caused by excessive administration, and thus, will be unsuitable from economical efficiency.

Furthermore, the felbinac-containing patch of the invention is such that the drug release rate of the patch at one hour after the start of test is 20±15 wt%, the drug release rate at three hours after the start of test is 40 ±15 wt%, and the drug release rate at six hours after the start of test is 60±20 wt%, when the water-releasing test using a rotating cylinder described in the release test method as prescribed in United State Pharmacopoeia is carried out under the following conditions:
Test solution: Solution 2 described in the disintegration test as prescribed in The Pharmacopoeia of Japan
Solution temperature: 37±0.5 °C
The distance between the lower end of cylinder and the inner surface of container: 12±2 mm
The number of rotations: 50 rpm.

If the drug release rate is below the lower limit of the above-mentioned range, sufficient efficacy will not be attained. On the other hand, if it exceeds the upper limit, that will raise concern for bringing harmful effects such as skin irritation caused by excessive administration of the drug. Accordingly, the felbinac-containing patch of the invention, which is able to stably maintain such constantly high drug release, has for the first time made it possible to provide a medicine that stably produces an excellent therapeutic effect over a long period of time without bringing harmful effects such as skin irritation upon administration.

The felbinac-containing patch of the present invention may be obtained by including felbinac as a medicinally effective component in a specific blending ratio into a base containing a styrene-isoprene-styrene block copolymer, a rosin-based resin, and a plasticizer in the respective blending proportions. Various base components used for forming the patch of the invention will be described in detail hereafter.

The styrene-isoprene-styrene block copolymer according to the present invention is a block copolymer of styrene and isoprene and is provided with polystyrene chains at both ends thereof. Such styrene-isoprene-styrene block copolymers include, among others, Krayton D-KX401CS and D-1107CU (trade names, available from Shell Kagaku K.K.); JSR SIS-5000 and 5002 (trade names, available from Japan Synthetic Rubber Co., Ltd.); Quintac 3530, 3421 and 3570C (trade names, available from Nippon Zeon Co., Ltd.); and Solprene 428 (trade name, available from Phillips Petroleum International Ltd.): one kind or a combination of two or more kinds of the foregoing may be used.

The blending ratio of the styrene-isoprene-styrene block copolymer is 10-30 wt%, preferably 15-25 wt%, based on the total weight of the patch. Here, if the blending ratio is less than 10 wt%, the cohesive force of the base will lower and shape retention of the base will degrade. On the other hand, if it exceeds 30 wt%, the cohesive force of the base will increase, thus resulting in a reduction in adhesive strength and lowering workability.

The polyisobutylene according to the present invention is a polymer of isobutylene. As such polyisobutylene, there are mentioned: Vistanex LM-MH, LM-H, MML-80, MML-100 and MML-120 (trade names, available from Exxon Chemical Japan Ltd.); Tetrax 4T, 5T and 6T (trade names, available from Nippon Petrochemicals Co., Ltd.); and Opanol B-30, B-50, B-100 and B-200 (trade names, available from BASF AG): one kind or a combination of two or more kinds of the foregoing may be used.

The blending ratio of the polyisobutylene is 5-15 wt%, preferably 5-10 wt%, based on the total weight of the patch. Here, if blending ratio is less than 5 wt%, the cohesive force of the base will lower and shape retention of the base will degrade. On the other hand, if it exceeds 15 wt%, the cohesive force of the base will increase, thus resulting in a reduction in adhesive strength and lowering workability.

The rosin-based resin according to the present invention is a resin containing rosin or a rosin derivative as a base material; and a rosin ester, a hydrogenated rosin ester, a maleic acid-modified rosin ester, or the like may preferably be used. Such rosin-based resins include, among others, Ester Gum A, AA-G, Hand HP (trade names, available from Arakawa Chemical Industries Ltd.); Hariester L, S and P (trade names, available from Harima Kasei Co., Ltd.); Pinecrystal KE-100 and KE-311 (trade names, available from Arakawa Chemical Industries Ltd.); Hercolyn D (trade name, available from Rika-Hercules Inc.); Foral 85 and 105 (trade names, available from Rika-Hercules Inc.); Stebelite Ester 7 and 10 (trade names, available from Rika-Hercules Inc.); and Pentalyn 4820 and 4740 (trade names, available from Rika-Hercules Inc.): one kind or a combination of two or more kinds of the foregoing may be used.

The blending ratio of the rosin-based resin is 5-30 wt%, preferably 10-25 wt%, based on the total weight of the patch. Here, if the blending ratio is less than 5 wt%, the adhesive strength will be reduced, causing prolonged adhesion on the skin to be difficult. On the other hand, if it exceeds 30 wt%, the percutaneous absorption and cohesiveness of the drug will be lowered.

The plasticizer according to the present invention is an agent compatible with the other base components and capable of providing the base with flexibility. Almond oil, olive oil, camellia oil, persic oil, peanut oil, olefinic acids, liquid paraffin, and the like may preferably be used. These plasticizers may be used singly or in a combination of two or more kinds, and among them, the liquid paraffin is particularly preferred.

The blending ratio of the plasticizer is 30-70 wt%, preferably 30-60 wt%, based on the total weight of the patch. Here, if the blending ratio is less than 30 wt%, the viscosity of an ointment will increase, causing nonuniformity of the ointment and lowering of workability. On the other hand, if it exceeds 70 wt%, the cohesive force of a preparation will be reduced, resulting in an increase in stickiness and so on proportionally.

In the felbinac-containing patch of the present invention, a dispersing agent is further contained in a specific blending ratio in the base containing the aforementioned styrene-isoprene-styrene block copolymer, polyisobutylene, rosin-based resin, and plasticizer. The dispersing agent according to the invention is an agent capable of enhancing dispersibility of different components in the preparation, particularly that of the styrene-isoprene-styrene block copolymer and of polyisobutylene. Synthetic aluminum silicate and/or hydrated aluminum silicate may preferably be used. These dispersing agents may be used singly or in a combination of two or more kinds, and among them, synthetic aluminum silicate is particularly preferred.

The blending ratio of the dispersing agent is 0.01-5 wt%, preferably 0.1-2 wt%, and more preferably 0.3-1 wt%, based on the total weight of the patch. Here, if the blending ratio is less than 0.01 wt%, the styrene-isoprene-styrene block copolymer and polyisobutylene will cohere to cause nonuniform dispersion, whereby the uniformity of felbinac is insufficient to attain the constant drug release. On the other hand, if it exceeds 5 wt%, interaction between the dispersing agent and felbinac will lower the drug release rate.

In the patch of the present invention, felbinac as a medicinally effective component ( i.e., 4-biphenylacetic acid) is contained in the above-mentioned base in a 1-5 wt% proportion as described above.

Because the dispersing agent is contained in the patch of the present invention having the above-stated composition, various components (particularly, the styrene-isoprene-styrene block copolymer and polyisobutylene) are uniformly dispersed in the preparation without cohesion and the uniform dispersibility of felbinac is adequately maintained. This will stably sustain the constantly high drug release in the patch of the present invention. Thus, the sufficient anti-inflammatory, analgesic effect by felbinac will be stably produced over a long period of time without bringing harmful effects such as skin irritation. Besides, the excellent stability and adhesion of preparations can be realized.

The patch of the present invention may further contain, if desired, another (other) additive component(s) such as an inorganic filler, a synthetic polymer, a tackifier, an antioxidant, an ultraviolet light absorber, an antihistamine, an antibacterial agent, or a perfume, in addition to the styrene-isoprene-styrene block copolymer, polyisobutylene, rosin-based resin, plasticizer, and felbinac as described above; among the foregoing, the patch preferably further contains the antioxidant.

Such other additive components include inorganic fillers (kaolin, titanium oxide, talc, zinc oxide, silica hydrate, magnesium carbonate, calcium hydrogenphosphate, magnesium silicate, diatomaceous earth, silicic anhydride, bentonite, etc.), synthetic polymers (polyacrylic polymers, synthetic polyisoprene rubbers, polystyrenes, polybutadiene rubbers, silicone rubbers, styrene-butylene-styrene block copolymers, styrene-isoprene block copolymers, etc.), tackifiers (terpene resins, petroleum resins, etc.), antioxidants (ascorbic acid, propyl gallate, butylhydroxyanisole, dibutylhydroxytoluene (BHT), nordihydroguaiaretic acid, tocopherol, tocopherol acetate, etc.), ultraviolet light absorbers (paraaminobenzoic acid, paraaminobenzoic ester, amyl paradimethylaminobenzoate, salicylic ester, methyl anthranilate, umbelliferone, esculin, benzyl cinnamate, cinoxate, guaiazulene, urocanic acid, 2-(2-hydroxy-5-methyphenyl) benzotriazole, 4-methoxybenzophenone, 2-hydroxy-4-methoxybenzophenone, octabenzone, dioxybenzone, dihydroxydimethoxybenzophenone, sulisobenzone, benzoresorsinol, octyl dimethylparaamino benzoate, ethylhexyl p-methoxy-cinnamate, etc.), antihistamines (isopentyl chloride, diphenhydramine hydrochloride, iproheptine hydrochloride, diphenylpyraline hydrochloride, cyproheptadine hydrochloride, triprolidine hydrochloride, promethazine hydrochloride, homochlorcyclizine hydrochloride, alimemazine tartrate, diphenhydramine tannate, diphenylpyraline piprinhydrinate, clemastine fumarate, chlorpheniramine maleate, dimethindene maleate, mequitazine, etc.), antibacterial agents (paraoxybenzoic ester, benzoic acid, benzoate, salicylate, sorbic acid, sorbate, dehydroacetate, 4-isopropyl-3-methylphenol, 2-isopropyl-5-methylphenol, hinokitiol, cresol, 2, 4, 4-trichloro-2'-hydroxydiphenylether, 3, 4, 4'-trichiorocarbanilide, chlorobutanol, benzalkonium chloride, benzethonium chloride, etc.), refrigerants or perfumes (1-menthol, etc.), and the like.

The blending ratio of such further additive component is preferably 0.01-7 wt%, more preferably 0.1-5 wt%, based on the total weight of the patch. Among these additive components, the blending ratio of the antioxidant is preferably 0.1-5 wt%, more preferably 0.5-2 wt%, based on the total weight of the patch. If the blending ratio is below the lower limit, the base will deteriorate with time, which tends to cause increases in the remainder of ointment, stickiness, and the like. On the other hand, if it exceeds the upper limit, the cohesive force of the preparation will lower and the shape retention of the preparation will degrade, which tends to cause increased pain upon stripping and more stickiness, and so on.

The thickness (not including thicknesses of a backing and an exfoliated liner described hereafter) of the patch (i.e., the plaster layer) of the present invention prepared using the above components is preferably 50-300 µm and more preferably 80-200 µm. Here, if the thickness is less than 50 µm, the persistence of adhesiveness and adhesion will tend to be reduced. On the other hand, if it exceeds 300 µm, the cohesive force will tend to lower and the shape retention will tend to degradee.

Because of the use of a highly flexible backing as described below, the patch of the present invention freely expands and contracts longitudinally and laterally, thus achieving the affixing feeling of high level.

The felbinac-containing patches of the present invention described above possess the following various excellent characteristics:
(1) Since felbinac that has an excellent anti-inflammatory, analgesic action exhibits constantly high release, and efficacy with great therapeutic effects is attained.
(2) Since a styrene-isoprene-styrene block copolymer, polyisobutylene, a rosin-based resin, a plasticizer, and a dispersing agent have been selected as base components, the constantly high drug release is maintained stably while the stability of the preparation will prove to be satisfactory; and a reduction in skin irritation is achieved.
(3) Since it has become possible to employ a backing with flexibility, adhesion is realized such that the patch can be affixed even to a flexion site like elbow or knee with sufficient compatibility and without being peeled off over a long period of time.

The backing of patch of the present invention is desirably one that does not affect release of the drug from the patch of the invention; and flexible or non-flexible materials may be used. Usable backings for the invention include, among others, a film, a sheet, a sheet porous body, a sheet foam, and a woven or non-woven fabric of a synthetic resin such as polyethylene, polypropylene, polybutadien, an ethylene-vinyl acetate copolymer, polyvinyl chloride, a polyester, nylon, or a polyurethane; paper; fabric; non-woven fabric; and a laminated product of the foregoing.

Among these backings for patches, a backing with flexibility is preferred. Its load at 30%-elongation (as set forth in JIS L 1096) is preferably 100-800 g longitudinally (in the direction of the long side) and 500 -2500 g laterally (in the direction of the short side), more preferably 100-500 g longitudinally and 500-2000 g laterally, under such measurement conditions that the sample width is 50 mm, the sample length 200 mm, and the elongation speed 200 mm/min. Also, its recovery factor at 50%-elongation (as set forth in JIS L 1096) is preferably 75-95% longitudinally and 65-85% laterally, more preferably 80-95% longitudinally and 70-85% laterally, under such measurement conditions that the sample width is 50 mm, the sample length 200 mm, and the elongation speed 200 mm/min. If the load at 30%-elongation for these backings is below the lower limit, the backing will lose its sturdiness when affixed and handling tends to be difficult. On the other hand, if it exceeds the upper limit, adhesion to a flexion site over a long period of time tends to be difficult. Further, if the recovery factor at 50%-elongation for those backings is below the lower limit, compatibility with the flexion site when affixed thereto will be poor and satisfactory adhesion is not likely to be attained. On the other hand, if it exceeds the upper limit, handling tends to be difficult upon affixing.

As described above, the patch of the present invention, through the uses of a highly flexible backing, can firmly be affixed to flexion sites with vigorous movement such as elbow and knee. Furthermore, the basic weight (weight per unit area) of the backing according to the invention is preferably 100±30 g/m².

A preferred example of the method for preparing the felbinac-containing patch of the present invention will be described below.

First, the styrene-isoprene-styrene block copolymer, polyisobutylene, rosin-based resin, plasticizer, and dispersing agent (and other additive components, etc. if any) are blended each in a predetermined ratio to yield a mixture, and the mixture is heated and stirred under an inert atmosphere of nitrogen or the like to yield a dissolved material. The temperature upon stirring is preferably 110-200 °C and the stirring time is preferably 30-120 minutes. Subsequently, felbinac (the medicinally effective component) is added to the above dissolved material and the mixture is then stirred preferably at 110-200 °C and preferably for 5-30 minutes, thereby yielding a uniformly dissolved material. Also, the various components described above may be added to an organic solvent such as toluene or ethyl acetate so as to give their predetermined proportions, and may be stirred to yield a uniformly dissolved material.

Next, this dissolved material is spread directly over the backing by an ordinary method, and thereafter, may be covered by a released liner (or peeling cover); or alternatively, once this dissolved material has been spread over the released liner, it is allowed to cover the backing and thus it may be pressed and transferred onto the backing. When a uniformly dissolved material has been obtained using an organic solvent, it is dried with a dryer, after having been spread over the backing, and the organic solvent is removed by evaporation, after which the dissolved material may preferably be pressed and transferred onto the backing. Such released liners include, among others, a released paper processed by release treatment (treatment for facilitating release); cellophane; and a plastic film of polyethylene, polypropylene, polyester, or the like.

It should be noted that only one embodiment has been described with respect to the order of blending the respective base components, the medicinally effective component, and the other additive components in the above preparation method and that the preparation method for the patch of the present invention is not limited to this method relying on the blending order.

### EXAMPLES

The felbinac-containing patches of the present invention will be described in more detail by way of examples and comparative examples; however, they are not to be limited to those described in the following examples. In the examples and comparative examples, "part(s)" mean "part(s) by weight" unless otherwise stated specifically.

### Example 1

| | |
|---|---|
| styrene-isoprene-styrene block copolymer (Krayton D KX401CS) | 21.0 parts |
| polyisobutylene | 7.0 parts |
| rosin-based resin (Foral 85) | 15.0 parts |
| liquid paraffin | 53.0 parts |
| synthetic aluminum silicate | 1.0 part |
| felbinac | 3.0 parts |

A patch was prepared in the above formulation according to the aforementioned preparation method. Specifically, the components other than felbinac in the above formulation were blended to yield a mixture and the mixture was heated and stirred under the nitrogen atmosphere to yield a dissolved material. Subsequently, felbinac, the medicinally effective component, was added to the dissolved material and the mixture was heated and stirred to yield a uniformly dissolved material. Then, this dissolved material was spread over a backing (non-woven fabric of polypropyrene) so that the thickness of the plaster layer obtained was 150 µm. Thereafter, the dissolved material was covered with a released liner (polyester film) and the product after cooled was cut into the desired size, whereby a felbinac-containing patch was obtained. The backing used here was one of which the load at 30%-elongation was 250 g longitudinally and 1200 g laterally and the recovery factor at 50%-elongation was 90% longitudinally and 75% laterally.

### Example 2

| | |
|---|---|
| styrene-isoprene-styrene block copolymer (SIS-5000) | 20.0 parts |
| polyisobutylene | 5.0 parts |
| rosin-based resin (Estergum H) | 16.0 parts |
| liquid paraffin | 55.8 parts |
| synthetic aluminum silicate | 0.2 part |
| felbinac | 3.0 parts |

A patch was prepared in the above formulation according to the aforementioned preparation method. Specifically, the components other than felbinac in the above formulation were blended to yield a mixture and the mixture was heated and stirred under the nitrogen atmosphere to yield a dissolved material. Subsequently, felbinac, the medicinally effective component, was added to the dissolved material and the mixture was heated and stirred to yield a uniformly dissolved material. Then, this dissolved material was spread over a backing (woven fabric made of polyester) so that the thickness of the plaster layer obtained was 150 µm. Thereafter, the dissolved material was covered with a released liner (polyester film) and the product after cooled was cut into the desired size, whereby a felbinac-containing patch was obtained. The backing used here was one of which the load at 30%-elongation was 500 g longitudinally and 1800 g laterally and the recovery factor at 50%-elongation was 80% longitudinally and 70% laterally.

### Example 3

| | |
|---|---|
| styrene-isoprene-styrene block copolymer (SIS-5000) | 18.0 parts |
| styrene-isoprene-styrene block copolymer (Krayton D KX401CS) | 3.0 parts |
| polyisobutylene | 5.0 parts |
| rosin-based resin (Pinecrystal KE-311) | 15.0 parts |
| liquid paraffin | 53.2 parts |
| dibutylhydroxytoluene | 1.0 parts |
| synthetic aluminum silicate | 1.8 part |
| felbinac | 3.0 parts |

A patch was prepared in the above formulation according to the aforementioned preparation method. Specifically, the components other than felbinac in the above formulation were blended to yield a mixture and the mixture was heated and stirred under the nitrogen atmosphere to yield a dissolved material. Subsequently, felbinac, the medicinally effective component, was added to the dissolved material and the mixture was heated and stirred to yield a uniformly dissolved material. Then, this dissolved material was spread over a backing (film made of polyurethane) so that the thickness of the plaster layer obtained was 150 µm. Thereafter, the dissolved material was covered with a released liner (polyester film) and the product after cooled was cut into the desired size, whereby a felbinac-containing patch was obtained. The backing used here was one of which the load at-30% elongation was 300 g longitudinally and 850 g laterally and the recovery factor at 50%-elongation was 85% longitudinally and 75% laterally.

### Example 4

| | |
|---|---|
| styrene-isoprene-styrene block copolymer (SIS-5000) | 10.5 parts |
| styrene-isoprene-styrene block copolymer (Krayton D KX401CS) | 10.5 parts |
| polyisobutylene | 5.0 parts |
| rosin-based resin (Stebelight Ester 7) | 14.2 parts |
| liquid paraffin | 55.0 parts |
| dibutylhydroxytoluene | 1.0 parts |
| synthetic aluminum silicate | 0.8 part |
| felbinac | 3.0 parts |

A patch was prepared in the above formulation according to the aforementioned preparation method. Specifically, the components other than felbinac in the above formulation were blended to yield a mixture and the mixture was heated and stirred under the nitrogen atmosphere to yield a dissolved material. Subsequently, felbinac, the medicinally effective component, was added to the dissolved material and the mixture was heated and stirred to yield a uniformly dissolved material. Then, this dissolved material was spread over a backing (non-woven fabric made of polyester) so that the thickness of the plaster layer obtained was 150 µm. Thereafter, the dissolved material was covered with a released liner (polyester film) and the product after cooled was cut into the desired size, whereby a felbinac-containing patch was obtained. The backing used here was one of which the load at 30%-elongation was 600 g longitudinally and 1800 g laterally and the recovery factor at-50% elongation was 90% longitudinally and 85% laterally.

### Example 5

| | |
|---|---|
| styrene-isoprene-styrene block copolymer (SIS-5000) | 4.0 parts |
| styrene-isoprene-styrene block copolymer (Krayton D KX401CS) | 15.0 parts |
| polyisobutylene | 5.93 parts |
| rosin-based resin (Stebelight Ester7) | 15.0 parts |
| liquid paraffin | 55.0 parts |
| dibutylhydroxytoluene | 1.0 parts |
| synthetic aluminum silicate | 0.07 parts |
| felbinac | 3.0 parts |

A patch was prepared in the above formulation according to the aforementioned preparation method. Specifically, the components other than felbinac in the above formulation were blended to yield a mixture and the mixture was heated and stirred under the nitrogen atmosphere to yield a dissolved material. Subsequently, felbinac, the medicinally effective component, was added to the dissolved material and the mixture was heated and stirred to yield a uniformly dissolved material Then, this dissolved material was spread over a backing (non-woven fabric made of polyester) so that the thickness of the plaster layer obtained was 150 µm. Thereafter, the dissolved material was covered with a released liner (polyester film) and the product after cooled was cut into the desired size, whereby a felbinac-containing patch was obtained. The backing used here was one of which the load at 30%-elongation was 600 g longitudinally and 1800 g laterally and the recovery factor at 50%-elongation was 90% longitudinally and 85% laterally.

### Comparative Example 1

| | |
|---|---|
| styrene-isoprene-styrene block copolymer (SIS-5000) | 18.0 parts |
| polyisobutylene | 5.0 parts |
| rosin-based resin (Pinecrystal KE-311) | 25.0 parts |
| liquid paraffin | 49.0 parts |
| felbinac | 3.0 parts |

A patch was prepared in the above formulation according to the aforementioned preparation method. Specifically, the components other than felbinac in the above formulation were blended to yield a mixture and the mixture was heated and stirred under the nitrogen atmosphere to yield a dissolved material. Subsequently, felbinac, the medicinally effective component, was added to the dissolved material and the mixture was heated and stirred to yield a uniformly dissolved material. Then, this dissolved material was spread over a backing (woven fabric made of polyester) so that the thickness of the plaster layer obtained was 150 µm. Thereafter, the dissolved material was covered with a released liner (polyester film) and the product after cooled was cut into the desired size, whereby a felbinac-containing patch was obtained. The backing used here was one of which the load at 30%-elongation was 290 g longitudinally and 1460 g laterally and the recovery factor at 50%-elongation was 82% longitudinally and 70% laterally.

### Comparative Example 2

| | |
|---|---|
| styrene-isoprene-styrene block copolymer (Krayton D KX401CS) | 19.0 parts |
| polyisobutylene | 6.0 parts |
| rosin-based resin (Pinecrystal KE-100) | 14.0 parts |
| liquid paraffin | 50.0 parts |
| dibutylhydroxytoluene | 2.0 parts |
| synthetic aluminum silicate | 6.0 parts |
| felbinac | 3.0 parts |

A patch was prepared in the above formulation according to the aforementioned preparation method. Specifically, the components other than felbinac in the above formulation were blended to yield a mixture and the mixture was heated and stirred under the nitrogen atmosphere to yield a dissolved material. Subsequently, felbinac, the medicinally effective component, was added to the dissolved material and the mixture was heated and stirred to yield a uniformly dissolved material. Then, this dissolved material was spread over a backing (woven fabric made of polyester) so that the thickness of the plaster layer obtained was 150 µm. Thereafter, the dissolved material was covered with a released liner (polyester film) and the product after cooled was cut into the desired size, whereby a felbinac-containing patch was obtained. The backing used here was one of which the load at 30%-elongation was 250 g longitudinally and 1200 g laterally and the recovery factor at 50%-elongation was 90% longitudinally and 75% laterally.

### Comparative Example 3

| | |
|---|---|
| sodium polyacrylate | 5.0 parts |
| sodium carboxymethyl cellulose | 5.0 parts |
| sorbitol | 15.0 parts |
| gelatin | 10.0 parts |
| titanium oxide | 5.0 parts |
| glycerol | 15.0 parts |
| purified water | 40.0 parts |
| felbinac | 5.0 parts |

A gel patch was prepared in the above formulation according to the aforementioned preparation method. Specifically, the components other than felbinac in the above formulation were blended, and then, felbinac, the medicinally effective component, was added to the mixture, yielding a uniform mixture. Then, this dissolved material was spread over a backing (woven fabric made of polyester) so that the thickness of the plaster layer obtained was 1000 µm. Thereafter, the dissolved material was covered with a released liner (polyester film) and the product after cooled was cut into the desired size, whereby a felbinac-containing gel patch was obtained. The backing used here was one of which the load at 30%-elongation was 250g longitudinally and 1200 g laterally and the recovery factor at 50%-elongation was 90% longitudinally and 75% laterally.

### Comparative Example 4

2-Ethylhexyl acrylate 96.5 parts, acrylic acid 3.0 parts, and benzoyl peroxide 0.5 parts were allowed to polymerize in ethyl acetate to yield an acrylic binder having a solid content of 50.0%. This acrylic binder was used to prepare a patch according to the following formulation:

| | |
|---|---|
| acrylic binder solution | 96.0 parts |
| dibutylhydroxytoluene | 1.0 parts |
| felbinac | 3.0 parts |

Specifically, various components in the above formulation were blended to yield a mixture and the mixture was heated and stirred to yield a dissolved material. Then, this dissolved material was spread over a released liner (polyester film) so that the thickness of the plaster layer obtained was 80 µm. Thereafter, ethyl acetate was evaporated for removal by drying with warm air and the dissolved material was covered with a backing (woven fabric made of polyester) and the product was cut into the desired size, whereby a felbinac-containing patch was obtained. The backing used here was one of which the load at 30%-elongation was 250 g longitudinally and 1200 g laterally and the recovery factor at 50%-elongation was 90% longitudinally and 75% laterally.

### Test Example 1 (drug release test)

A test for the drug release capabilities of the patches obtained in Examples 1, 2, 3, 4, and 5 and Comparative Examples 2 and 4 was carried out according to the rotating cylinder method described in the release test as prescribed in United State Pharmacopoeia (USP) under the following conditions:
Test solution: Solution 2 described in the disintegration test method as prescribed in The Pharmacopoeia of Japan
Solution temperature: 37±0.5 °C
The distance between the lower end of cylinder and the inner surface of container: 12±2 mm
The number of rotations: 50 rpm.

The results obtained are shown in Fig. 1. The horizontal axis represents the time after the start of test, while the vertical axis represents the release rate of felbinac.

As is apparent from the results shown in Fig. 1, the drug release rates of patches of the present invention as obtained in Examples 1, 2, 3, 4, and 5 at one hour after the start of test were 23.5%, 23.8%, 24.5%, 21.0%, and 18.0%, respectively; those at three hours after the start of test were 40.7%, 45.0%, 47.0%, 38.0%, and 35.0%, respectively; and those at six hours after the start of test were 59.4%, 66.1%, 68.0%, 57.0%, and 52.0%, respectively. It was thus ascertained that said patches stably maintained constantly high drug release.

By contrast, the patch containing 6.0 wt% of a dispersing agent as obtained in Comparative Example 2 and the patch with an acrylic binder as obtained in Comparative Example 4 both had low drug release rates; and moreover, it was ascertained that their drug release rates even decrease as time lapsed.

### Test Example 2 (patch-sensing test)

The patches obtained in Example 1 and Comparative Example 3 were used to perform the patch-sensing test in which 30 healthy male adults were subjected. Specifically, each patch (10x14 cm in size) was affixed to the knees of each subject; and the conditions of peeling about 8 hours later as well as the sense of fitting during the affixing period was evaluated. The results obtained are shown in Tables 1A and 1B.

**Table 1A**

| adhesion (the number of applicable subjects) | | | | | |
|---|---|---|---|---|---|
| | peeling ≧ 1/2 | peeling ≧ 1/3 | peeling ≧ 1/4 | partial peeling of corner | no peeling at all |
| Ex. 1 | 0 | 0 | 1 | 7 | 22 |
| Com.Ex.3 | 1 | 3 | 11 | 16 | 0 |
| Ex.: Example, Com.Ex.: Comparative Example | | | | | |

**Table 1B**

| sense of fitting (the number of applicable subjects) | | | | | |
|---|---|---|---|---|---|
| | no fitting at all | almost no fitting | some fitting | fitted | well fitted |
| Ex. 1 | 0 | 0 | 3 | 9 | 18 |
| Com.Ex.3 | 7 | 11 | 12 | 0 | 0 |
| Ex.: Example, Com.Ex.: Comparative Example | | | | | |

As is apparent from the results shown in Tables 1A and 1B, the patch obtained in Example 1 was highly evaluated both in the adhesion properties and in the sense of fitting; and it was confirmed to be a very excellent patch for use. By contrast, the patch with aqueous bases obtained in Comparative Example 3 had poor adhesive properties over a long period of time, nor had satisfactory sense of fitting.

### Test Example 3 (skin irritation test)

The patches obtained in Example 2, 3, and 4 and Comparative Example 4 were used to perform the 48-hours patch test in which 30 healthy male adults were subjected for the evaluation of skin irritation. Specifically, each patch (size: circle with a diameter of 2.5 cm) was affixed to the back of each subject, and the skin irritation index about 48 hours later was obtained. Here, the skin irritation index was calculated according to the Sugai equation ("Skin" vol. 27, No. 4, August Issue, 1985). The results obtained are shown in Table 2.

**Table 2**

| human skin irritation test results | |
|---|---|
| | skin irritation index |
| Example 2 | 12.0 |
| Example 3 | 13.2 |
| Example 4 | 15.0 |
| Comparative Example 4 | 32.8 |
| When applied to healthy skin 15.0 or less: safe product 15.0-30.0: permissible product 30.0-60.0: product to be improved 60.0 or more: dangerous product | |

As is apparent from the results shown in Table 2, the patches of the present invention as obtained in Examples 2, 3, and 4 had skin irritation indices of 15 or less, which were low sufficient to ascertain their great safety. By contrast, the patch with an acrylic binder obtained in Comparative Example 4 had a skin irritation index of 32.8 or more, which was high sufficient to ascertain their strong skin irritation.

### Test Example 4 (stability test of adhesive strength)

The patches obtained in Examples 1, 3, and 4 and Comparative Examples 1 and 3 were used to evaluate the stability of their adhesive strengths according to the probe tack test method. Specifically, the probe tack test method as set forth in ASTM D 2979 was followed: each patch was stored under the condition of 40 °C; and the adhesive strength of each patch was determined by the adhesive strength measuring method as set forth in ASTM D 2979 at the initial stage, after 3-month storage, and after 6-month storage. The results obtained are shown in Table 3.

**TABLE 3**

| | probe tack adhesive strength stability test (g) | | |
|---|---|---|---|
| | initial value | after storage 40 °C,3 months | after storage 40 °C,6 months |
| Example 1 | 54.3 | 52.4 | 55.3 |
| Example 3 | 55.7 | 55.9 | 53.1 |
| Example 4 | 50.2 | 49.5 | 49 |
| Comparative Example 1 | 51.5 | 42.8 | 38.5 |
| Comparative Example 3 | 27.8 | 24.8 | 20.5 |

As is apparent from the results shown in Table 3, the patches obtained in Examples 1, 3, and 4 showed almost no change in adhesive strength after the accelerated test; and it was ascertained that the stability of adhesive strength was very high. By contrast, the patch obtained in Comparative Example 1 containing no dispersing agent showed a time-dependent decrease in adhesive strength; and the stability of its adhesion strength was confirmed to be low. Moreover, the patch with aqueous bases obtained in Comparative Example 3 had low adhesive strength even when initially prepared and, as a result, it was not satisfactory.

As described above, the present invention can provide a felbinac-containing patch that stably produces a sufficient anti-inflammatory, analgesic effect by felbinac exhibiting a strong anti-inflammatory, analgesic action without bringing harmful effects such as skin irritation over a long period and, at the same time, that is excellent in the stability of preparations as well as in their adhesive properties. Accordingly, the present invention can provide the felbinac-containing patch (plaster) that is useful as a medicinal preparation (patch for external application) which is intended for anti-inflammatory, analgesic effect.

From the invention thus described, it will be obvious that the invention may be varied in many ways. Such variations are not to be regarded as a departure from the spirit and scope of the invention, and all such modifications as would be obvious to one skilled in the art are intended for inclusion within the scope of the following claims.

## Claims

1. A felbinac-containing patch comprising 1-5 wt% of felbinac as a medicinally effective component,
wherein the drug release rate of the patch at one hour after the start of test is 20±15 wt%, the drug release rate of the patch at three hours after the start of test is 40 ±15 wt%, and the drug release rate of the patch at six hours after the start of test is 60±20 wt%, when the water-releasing test using a rotating cylinder described in the release test method as prescribed in United State Pharmacopoeia is carried out under the following conditions:
Test solution: Solution 2 described in the disintegration test method as prescribed in The Pharmacopoeia of Japan
Solution temperature: 37±0.5 °C
The distance between the lower end of cylinder and the inner surface of container: 12±2 mm
The number of rotations: 50 rpm.

2. The felbinac-containing patch according to claim 1, wherein the patch comprises 10-30 wt% of a styrene-isoprene-styrene block copolymer, 5-15 wt% of polyisobutylene, 5-30 wt% of a rosin-based resin, 30-70 wt% of a plasticizer, 1-5 wt% of felbinac, and 0.01-5 wt% of a dispersing agent.

3. The felbinac-containing patch according to claim 2, wherein the dispersing agent is synthetic aluminum silicate and/or hydrated aluminum silicate.

4. The felbinac-containing patch according to claim 1, wherein the patch is further provided with a backing, and the backing has a load at 30 %-elongation of 100-800 g in the longitudinal direction and a load at 30 %-elongation of 500-2500 g in the lateral direction as well as has a recovery factor at 50%-elongation of 75-95% in the longitudinal direction and a recovery factor at 50%-elongation of 65-85% in the lateral direction, under such measurement conditions that the sample width is 50 mm, the sample length is 200 mm, and the elongation speed is 200 mm/min.
